# EUROPÄISCHE PATENTSCHRIFT

(11) **EP 0 782 845 B1**
(45) Veröffentlichungstag und Bekanntmachung des Hinweises auf die Patenterteilung: **11.09.2002**
(21) Anmeldenummer: 96102822.2
(22) Anmeldetag: 26.02.1996
(51) Int. Cl.: A61K 7/00

(54) **Haarfärbemittel und Haarfärbemittelbrei zum Färben von Humanhaaren**
Hair colorant and hair colorant slurry for dyeing human hair
Teinture pour cheveux et pâte de teinture pour cheveux pour teinter des cheveux humains

(30) Priorität: 05.01.1996 DE 19600221
(43) Veröffentlichungstag der Anmeldung: 09.07.1997
(73) Patentinhaber: Hans Schwarzkopf GmbH & Co. KG, 22763 Hamburg (DE)
(72) Erfinder: Akram, Mustafa, Dr., 22457 Hamburg (DE); Wolff, Wolfgang, 22941 Bargteheide (DE); Schlagenhoff, Sandra, 22763 Hamburg (DE); Schwartz, Stephan, 22880 Wedel (DE); Kleen, Astrid, Dr., 22081 Hamburg (DE)
(74) Vertreter: Foitzik, Joachim Kurt

(56) Entgegenhaltungen:
- DE-A- 4 233 874
- FR-A- 2 510 401
- US-A- 4 183 366
- US-A- 4 275 055
- US-A- 4 658 839
- Römpp Chemie Lexikon, 9. Auflage, Georg Thieme Verlag Stuttgart, Paperback-Ausgabe 1995

## Beschreibung

Die Erfindung betrifft Haarfärbemittel und Haarfärbemittelbrei.

Die Verwendung von Pflanzenfarbstoffen als Haarfärbemittel ist lange bekannt (Vergl. Edward Sagarin, Cosmetic Science and Technology, Interscience Publishers, Inc, New York 1957, Seiten 489-495; Janistyn, Handbuch der Kosmetika und Riechstoffe, Dr. Alfred Huthig Verlag, Heidelberg, 1973, Seiten 378-418). Der gravierende Nachteil dieses bekannten Färbens von Haaren mit Pflanzenfarben besteht darin, daß man keine ausreichende Abdeckung der grauen Haaren erzielen kann.

In der FR-OS 1583606 ist ein 2-Schrittverfahren zum Färben von Haaren beschrieben, bei dem in einem ersten Schritt ein nicht-oxidierender selbstziehender Farbstoff, nämlich Hennapulver, zur Einwirkung gebracht wird und in einem zweiten Schritt ein heißer Auszug Zwiebelschalen für 5 Minuten zur Einwirkung gebracht wird, wobei graue Humanhaare eine kupferartige Färbung erhalten sollen.

In der BE-OS 900219 sind Haarfärbemittel, die Pflanzenextrakte enthalten, beschrieben, die ebenfalls in einem 2-Schrittverfahren zum Färben von Haaren Verwendung finden, wobei im ersten Schritt das Haar mit einer wäßrigen Lösung, die zwei verschiedene Typen von oberflächenaktiven Mitteln und mindestens ein Metallsalz enthält, und in einer zweiten Stufe mit einer Lösung oder Dispersion von Farbstoffen (Curcuma), die die beiden Typen der oberflächenaktiven Mittel enthält, behandelt wird, wodurch das Haar einen Goldton erhält

In der GB-OS 2190104 ist ebenfalls ein Zweistufenverfahren zum Färben von Haaren beschrieben; hierbei werden ebenfalls zwei verschiedene Lösungen (A) und (B) verwendet. Die Lösungen (A) und (B) können z B in beliebiger Reihenfolge 3 bis 30 Minuten im Haar verbleiben. Hierzu kann die Lösung (A) im ersten Schritt als nicht-oxidierende selbstziehende Farbstoffe ein Kupfer(II)salz, Nitrobenzolderivate, Anthrachinon oder Azoverbindungen enthalten. Die Lösung (B) im zweiten Schritt kann als Naturprodukt Brazilin oder Hämatoxylin und andere natürliche Farbstoffe (z.B. Lawsonia Henna) enthalten.

In der DE-PS 3829102 wird ein zweistufiges Verfahren zum Färben von Haaren mit Pflanzenfarbstoffen und direktziehenden Farbstoffen zur Abdeckung der grauen Haare beschrieben, bei dem im ersten Schritt ein nicht-oxidierender selbstziehender Farbstoff für die Dauer von 3 bis 20 Minuten bei einer durch Anfeuchten und Erwärmen bewirkten Quellung des Haares zur Einwirkung gebracht wird, und danach in einem zweiten Schritt ein neben Lawsonia Henna ggf. andere pflanzliche Farbstoffe enthaltener Pflanzenbrei auf die Haare aufgetragen und 10 bis 40 Minuten zur Einwirkung gebracht wird.

Alle diese Haarfärbemittel können den genannten Nachteil der mangelnden Grauabdeckung nicht beseitigen.

Aufgabe der Erfindung ist es, Haarfärbemittel und Haarfärbemittelbrei zur Verfügung zu stellen, mit denen es gelingt, beim Färben der Haare eine sichere Grauabdeckung unter Schonung der Haarstruktur zu erzielen. Ferner sollen die Färbeergebnisse reproduzierbar und in einfachster Weise unter Vermeidung von Dosierfehlern erfolgen.

Diese Aufgabe wird durch die Haarfärbemittel gemäß Patentansprüche 1 bis 18 vollständig gelöst.

Hervorzuheben ist, daß die Haarfärbemittel der Erfindung, obwohl auf Basis von Oxidationsfarbstoffvorprodukten und Oxidationsmittel aufgebaut, die bekanntlich bei der Verwendung die Haarstruktur stark angreifen, den großen Vorteil besitzen, sich bei ihrer Verwendung als außerordentlich schonend für die behandelten Haare im Vergleich mit den Behandlungsergebnissen von Haarfärbemitteln nach Stand der Technik zu erweisen.

Der Gehalt an Pulver von farbstoffhaltigen Pflanzenteilen in den erfindungsgemäßen Haarfärbemitteln kann 3 bis 25 Gew-%, vorzugsweise 5 bis 15 Gew-%, bezogen auf das Gesamtgewicht des Haarfärbemittels betragen. Dieses Pflanzenpulver erhält man durch Mahlen und nachfolgendes Sieben von verschiedenen Pflanzenteilen wie Stengel, Blüten, Blätter, Wurzeln, Früchte, Samen usw. Vorzugsweise erhält man das Pflanzenpulver aus Kamille, kleinen Kamille-, Hibiskus-, Safor- und Sonnenblumenblüten; aus den Blättern des Walnussbaumes, des Hennastrauches, der Indigopflanze, des Sumachs und des Holunders, aus Rubiaceenwurzeln (Färberröte, Waldmeister, Labkraut), Curcuma-Wurzeln, Lotuswurzeln und Rhabarberwurzeln; aus rotem Sandel-, Campeche-, Brasilien- und Pernambucohölzern, aus Erlen-, Erdbeerbaum- und Sumarinde; aus Oleandersamen; aus Safranblütennarben; aus Curcumaund Blutkrautwurzelstöcken; aus Schlehenfrüchten, aus Ginsterzweigen und aus den ganzen Pflanzen von Wau, Flechten und der Goldrute. Die hier aufgezählten Pflanzen enthalten bekanntlich Farbstoffe und können in den erfindungsgemäßen Haarfärbemitteln bevorzugt als direktziehende Farbstoffe verwendet werden.

Traditionell besteht Henna aus den getrockneten, gepulverten Blättern der Pflanzen *Lawsonia alba*, *Lawsonia spinosa* und *Lawsonia inermis,* die von dem Hennastrauch gepflückt werden. Sogenanntes Henna schwarz leitet sich von den Blättern der Färberwaid (*Isatis tinctoria*) ab und wird in Mischungen mit *Lawsonia* Henna verwendet, um dunklere Schattierungen im Rotbereich zu erzielen.

Ferner gibt es Henna neutral, welches keinen aktiven Farbstoff enthält und welches zum Konditionieren von Haaren verwendet wird, wenn keine Färbung erfolgen soll. Henna neutral soll gemäß den Angaben in der US-PS 4 183 366, Spalte 1, Zeilen 16 bis 17 von den Blättern des Lotusbaumes stammen. Nach Angaben von Lieferfirmen von Henna neutral handelt es sich bei diesem Produkt um gepulverte Blätter und/oder Rinde von *Cassia auriculata.*

Über die Verwendung von Henna rot und Henna neutral bei der Färbung bzw. Konditionierung von Haaren berichtet Soap, Cosmetics Chemical Specialities, Volume 63, No. 11, Nov. 1987, Seite 36 bis 37, linke Spalte, Absätze 1 bis 4.

In den Beispielen 3 und 5 von DE 4233874 A1 sind Haartönungsmittel in Granulatform beschrieben, die neben Walnußschalenmehl bzw. Blauholzpulver, Kamillepulver, 1-(2-Hydroxyethyl)-amino-2-nitro-4-[bis-(2-hydroxyethyl)]-aminobenzol, 1,4-Dihydroxy-5,8-bis-[(2-hydroxyethyl)-amino]-antraquinon, Henna rot gepulvert und Henna neutral gepulvert enthalten. Henna rot und Henna neutral liegen a. a. O. somit nur mit Direktziehern auf pflanzlicher oder pflanzlicher und synthetischer Basis in Kombination vor.

Als Beispiele für einzusetzende Entwicklerkomponenten sind primäre aromatische oder heteroaromatische Amine mit einer weiteren in p-Stellung befindlichen funktionellen Gruppe, wie p-Phenylendiamin, p-Toluylendiamin, p-Aminophenol, 3-Methyl-4-aminophenol, 2-Hydroxyethyl-1,4-diaminobenzol, 2,4,5,6-Tetraaminopyrimidin, 2,5-Diaminopyridin und ihre Derivate, weitere Verbindungen der genannnten Art, die zusätzlich eine oder mehrere funktionelle Gruppen, wie OH-Gruppen, NH₂-Gruppen, NHR-Gruppen oder NRR-Gruppen tragen, wobei R einen gegebenenfalls substituierten Alkylrest mit 1 bis 4 Kohlenstoffatomen darstellt, anzuführen. Ferner können weitere, dem Fachmann bekannte Entwicklerkomponenten Verwendung finden. Kupplerkomponenten, wie z.B. α-Naphthol, Resorcin, 4-Chlorresorcin, 2-Methylresorcin, m-Aminophenol, m-Phenylendiamin, m-Toluylendiamin, 1,5- bzw. 1,7-Dihydroxynaphthalin, 5-Amino-2-methylphenol, 6-Amino-2-methylphenol bzw. Derivate der genannten Verbindungen, können eingesetzt werden. Auch weitere dem Fachmann bekannte Kupplerkomponenten können eingesetzt werden. Darüber hinaus können die Haarfärbemittel gegebenenfalls übliche direktaufziehende Farbstoffe enthalten, falls dies zur Erzielung gewisser Farbnuancen erforderlich ist.

Neben Henna neutral können Tannine und/oder Gerbsäuren (Gerbstoffe) in gepulverter Form der Pflanzenteile oder Pflanzenteilextrakten in Pulverform in Mengen von 1 bis 30 Gew.-% mitverwendet werden. Hierzu zählen Gerbstoffe pflanzlichen Ursprungs aus verschiedenen Pflanzenteilen wie z.B. Blättern (schwarzer Tee), Samen (Kaffee), Beeren (Rotwein), Gallen (vergl. Römpp Chemie Lexikon, 9. Auflage, Georg Thieme Verlag Stuttgart, Band 2, Paperback-Ausgabe 1995). Tannine sind in dem Buch Römpp Chemie Lexikon, 9. Auflage, Band 6 (Paperback-Ausgabe 1995) auf Seite 4452, rechte Spalte bis Seite 4453, linke Spalte, beschrieben. Die Art und Menge von mitzuverwendenden Tanninen und/oder Gerbsäuren (Gerbstoffen) kann an den gewünschten Farb- und Struktur-Effekt des zu färbenden Haares angepaßt werden.

In Haarfärbemitteln der Erfindung müssen mindestens 25 Gew-% Henna neutral enthalten sein, jedoch der Wert 70 Gew-% sollte nicht überschritten werden. Ein bevorzugter Bereich des Gehaltes an Henna neutral liegt zwischen 40 und 65 Gew-%, der bevorzugteste Bereich des Gehaltes an Henna neutral liegt bei 50 bis 60 Gew-%.

Als Oxidationsmittel kommen Additionsverbindungen von Wasserstoffperoxid am Harnstoff, Melamin oder Kalium-, Natriumborat sowie Gemische aus derartigen Wasserstoffperoxid-Anlagerungsverbindungen mit Kalium-, Natrium- oder Ammoniumperoxodisulfat in Betracht.

Zum Entstauben des pulverförmigen Haarfärbemittels wird inerte, nicht flüchtige, organische Flüssigkeit und/oder bei etwa 35 °C bis 50 °C schmeltende Wachse oder wachsartige, inerte Stoffe aufgesprüht. Dafür kommen im Prinzip alle pflanzlichen und tierischen Öle wie auch synthetische Öle und/oder Siliconöle in Betracht, beispielsweise Paraffinöl, Paraffin mit Schmelzpunkten im Bereich von 40 °C bis 50 °C und/oder Dimethyl-Polysiloxan und/oder Capryl-Caprinsäure-Triglycerid und/oder Octyldodecanol und/oder Bienenwachs.

Der Anteil der Öle bzw. Wachse in den erfindungsgemäßen Haarfärbemitteln kann zwischen 5 und 25 Gew-%, insbesondere zwischen 7 und 15 Gew-%, jeweils berechnet auf die Gesamt zusammensetzung des Haarfärbemittels, betragen. Die erfindungsgemäßen Haarfärbemittel können Guarmehl, Natriumalginat, Gummi arabicum, Guar- oder Caruba-Gummi, Pektine, Cellulosederivate wie auch Methylcellulose und Hydroxymethylcellulose, Hydroxypropylmethylcellulose, Carboxymethylcellulose und verschiedene Polymere, wie insbesondere Acrylsäurederivate, enthalten. Man kann auch anorganische Verdickungsmittel, wie Bentonit und/oder Kieselsäure, verwenden. Diese Verdickungsmittel sind vorzugsweise in Mengen zwischen 0,1 und 5 Gew-% und insbesondere zwischen 0,5 und 3 Gew-%, bezogen auf das Gesamtgewicht des Haarfärbemittels, enthalten.

Man kann den erfindungsgemäßen Haarfärbemitteln auch alle anderen herkömmlicherweise in Haarfärbemitteln verwendeten Adjuvantien zusetzen, wie insbesondere Penetrationsmittel, Sesquestriermittel, Antioxidantien, Puffer und Parfums.

Die Haarfärbemittel können auch anionische, nichtionische, kationische, amphotere Polymere, oberflächenaktive Mittel, oder Mischungen davon in Mengen von 0,1 bis 5 Gew-% enthalten.

Unter den bevorzugten oberflächenaktiven Mitteln kann man insbesondere nennen die Seifen, Alkylbenzolsulfonate, Alkylnaphthalinsulfonate, Fettalkoholsulfate oder - ethersulfate bzw. - sulfonate, quarternäre Ammoniumsalze, Fettsäurediethanolamide, polyethoxylierte oder polyglycerierte Alkylphenole. Die oberflächenaktiven Mittel liegen im erfindungsgemäßen Haarfärbemittel in Anteilen zwischen 0,0 und 25 Gew-%, vorzugsweise 0,1 und 15 Gew-%, bezogen auf das Gesamtgewicht des Färbemittels, vor. Der pH-Wert der erfindungsgemäßen Haarfärbemittel kann je nachdem mit organischen oder anorganischen Säuren, mit Hydroxiden, Alkali- oder Erdalkalicarbonaten oder Alkalisilikaten eingestellt werden.

Es ist überraschend, daß die konditionierende Wirkung von Henna neutral an dem gefärbten Haar voll erhalten wird, obwohl das Haarfärbemittel der Erfindung auch Oxidationsfarbstoffvorprodukte und Oxidationsmittel enthält.

Bei Verwendung des Haarfärbemittels soll das Haar trocken, vorher nicht gewaschen vorliegen. Für schulterlanges Haar wird 80 g Haarfärbemittel der Erfindung mit 320 ml heißem Wasser (etwa 50°C) zu einem streichfähigen Brei mit einem Schneebesen verarbeitet. Der erhaltene Haarfärbemittelbrei wird sofort mit einem Pinsel zügig und schnell vom Haaransatz bis zur Spitze satt aufgelegt. Das feine Schläfenhaar wird zuletzt nur dünn versorgt. Es ist darauf zu achten, daß das gesamte Haar gut mit dem Haarfärbemittelbrei umhüllt ist. Danach wird das gesamte Haar mit einer Haube so bedeckt, daß diese an den Konturen eng anliegt und so gebunden ist, daß diese nicht verrutschen kann. Auf dem so abgedeckten Haar wird 30 Minuten unter Wärmezufuhr (ca. 40°C) der aufgetragene Haarfärbemittelbrei belassen. Nach dieser Einwirkzeit wird die Haube abgenommem und der Haarfärbemittelbrei mit Leitungswasser (30°C) gründlich ausgeschwemmt. Danach wird das Haar shampooniert und gespült, bis Haar und Haut völlig sauber vorliegen.

Bevorzugte Ausführungsformen der Erfindung gemäß Patentanspruch 1 werden durch die folgenden Beispiele 1 bis 5 verdeutlicht, ohne jedoch die Erfindung auf die Beispiele zu beschränken.

### Beispiel 1

### Haarfärbemittel in Pulverform

| | |
|---|---|
| 0,10 kg | Resorcin |
| 0,10 kg | 1-Hydroxy-3-aminobenzol |
| 0,15 kg | 2-Amino-3-hydroxypyridin |
| 0,10 kg | 4-Hydroxypropylamino-3-nitrophenol |
| 0,15 kg | HC Yellow No.12 |
| 0,35 kg | 2-Methylresorcin |
| 1,20 kg | 2,5-Diaminotoluol Sulfat |
| 1,00 kg | Indigo |
| 2,00 kg | Walnußschalen |
| 4,00 kg | Henna rot |
| 54,85 kg | Henna neutral |
| 23,00 kg | Natriumperborat |
| 5,00kg | Carboxymethylcellulose |

92,00 kg der vorstehenden Mischung werden in einem Mischer (zum Beispiel einem Lödige-Mischer) zusammengerührt und anschließend mit 8,00 kg Paraffinöl (35 mPa*s, gemessen bei 20 °C) besprüht.

### Herstellung eines Haarfärbemittelbreis

40 g des Haarfärbemittels werden mit 160 g warmen Wassers (40°-50°C) gut vermischt, um einen gut streichfähigen Brei zu erhalten. Dieses Gemisch, mit einem Pinsel aufgetragen, läßt man 30 Minuten bei 40°C auf hellbraunes Haar mit einem Grauanteil von 50% einwirken. Danach spült man den Haarfärbemittelbrei aus, shampooniert und trocknet das Haar Das hellbraune Ausgangshaar mit einem Grauanteil von 50% zeigt einen einheitlichen hellen Mokkabraunton.

### Beispiel 2

### Haarfärbemittel in Pulverform

| | |
|---|---|
| 0,15 kg | Resorcin |
| 0,10 kg | 1-Hydroxy-3-aminobenzol |
| 0,15 kg | 2-Amino-3-hydroxypyridin |
| 0,15 kg | 4-Hydroxypropylamino-3-nitrophenol |
| 0,10 kg | HC Yellow No.12 |
| 0,30 kg | 2-Methylresorcin |
| 1,25 kg | 2,5-Diaminotoluol Sulfat |
| 1,00 kg | Kamille |
| 1,50 kg | Henna schwarz |
| 2,50 kg | Henna rot |
| 60,80 kg | Henna neutral |
| 20,00 kg | Natriumperborat |
| 5,00kg | Carboxymethylcellulose |

93,00 kg der vorstehenden Mischung werden in einem Mischer (zum Beispiel einem Lödige-Mischer) zusammengerührt und anschließend mit 7,00 kg Paraffinöl (15 mPa*s, gemessen bei 20 °C) besprüht.

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf dunkelblondes Haar mit einem Grauanteil von 50% einwirken. Danach spült man den Haarfärbemittelbrei aus, shampooniert und trocknet das Haar. Das dunkelblonde Ausgangshaar mit einem Grauanteil von 50% zeigt nach der Behandlung einen einheitlichen hellen Kastanienton.

### Beispiel 3

### Haarfärbemittel in Pulverform

| | |
|---|---|
| 0,30 kg | Resorcin |
| 0,10 kg | 1-Hydroxy-3-aminobenzol |
| 0,15 kg | 2-Amino-3-hydroxypyridin |
| 0,15 kg | HC Yellow No. 12 |
| 0,45 kg | 2-Methylresorcin |
| 2,00 kg | 2,5-Diaminotoluol Sulfat |
| 1,50 kg | Kamille |
| 2,00 kg | Faulbaumrinde |
| 3,50 kg | Henna rot |
| 52,85 kg | Henna neutral |
| 22,00 kg | Natriumperborat |
| 5,00kg | Carboxymethylcellulose |

90,00 kg der vorstehenden Mischung werden in einem Mischer (zum Beispiel einem Lödige-Mischer) zusammengerührt und anschließend mit 10,00 kg Capryl-Caprinsäure Triglycerid besprüht.

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf mittelbraunes Haar mit einem Grauanteil von 50% einwirken. Danach spült man den Haarfärbemittelbrei aus, shampooniert und trocknet das Haar. Das mittelbraune Ausgangshaar mit einem Grauanteil von 50% weist nach der Behandlung einen einheitlichen dunklen Mokkabraunton auf.

### Beispiel 4

### Haarfärbemittel in Pulverform

| | |
|---|---|
| 0,35 kg | Resorcin |
| 0,10 kg | 1-Hydroxy-3-aminobenzol |
| 0,25 kg | 2-Amino-3-hydroxypyridin |
| 0,30 kg | 4-Hydroxypropylamino-3-nitrophenol |
| 0,10 kg | HC Yellow No.12 |
| 0,45 kg | 2-Methylresorcin |
| 2,25 kg | 2,5-Diaminotoluol Sulfat |
| 1,00 kg | Blauholz |
| 1,00 kg | Indigo |
| 1,00 kg | Walnußschalen |
| 57,20 kg | Henna neutral |
| 22,00 kg | Natriumperborat |
| 5,00kg | Carboxymethylcellulose |

91,00 kg der vorstehenden Mischung werden in einem Mischer (zum Beispiel einem Lödige-Mischer) zusammengerührt und anschließend mit 9,00 kg Octyldodecanol besprüht.

### Herstellung eines Haarfärbemittelbreis

40 g des Haarfärbemittels werden mit 160 g warmen Wassers (40°-50°C) gut vermischt, um einen gut streichfähigen Brei zu erhalten. Dieses Gemisch, mit einem Pinsel aufgetragen, läßt man 30 Minuten bei 40°C auf hellbraunes Haar mit einem Grauanteil von 50% einwirken. Danach spült man den Haarfärbemittelbrei aus, shampooniert und trocknet das Haar. Das hellbraune Ausgangshaar mit einem Grauanteil von 50% weist einen einheitlichen dunklen Kastanienton auf.

### Beispiel 5

### Haarfärbemittel in Pulverform

| | |
|---|---|
| 0,20 kg | Resorcin |
| 0,15 kg | 1-Hydroxy-3-aminobenzol |
| 0,01 kg | 4-Hydroxypropylamino-3-nitrophenol |
| 0,05 kg | 2-Amino-6-chlor-4-nitrophenol |
| 0,40 kg | 2-Methylresorcin |
| 0,90 kg | 2,5-Diaminotoluol Sulfat |
| 1,50 kg | Blauholz |
| 1,00 kg | Kamille |
| 6,00 kg | Henna rot |
| 56,29 kg | Henna neutral |
| 21,00 kg | Natriumperborat |
| 5,00kg | Carboxymethylcellulose |

92,50 kg der vorstehenden Mischung werden in einem Mischer (zum Beispiel einem Lödige-Mischer) zusammengerührt und anschließend mit 7,50 kg Dimethyl-Polysiloxan besprüht

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf mittelblondes Haar mit einem Grauanteil von 80% einwirken. Danach spült man den Haarfärbemittelbrei aus, shampooniert und trocknet das Haar. Das mittelblonde Ausgangshaar mit einem Grauanteil von 80% liegt nach der Behandlung in einem einheitlichen Mittelblondton vor.

Die bervorzugtesten Ausführungsformen der Erfindung nach Anspruch 2 werden durch die folgenden Beispiele 6 bis 10 verdeutlicht, ohne jedoch die Erfindung auf die Beispiele zu beschränken.

### Beispiel 6

### Haarfärbemittel in Pulverform

| | |
|---|---|
| 0,10 kg | Resorcin |
| 0,10 kg | 1-Hydroxy-3-aminobenzol |
| 0,15 kg | 2-Amino-3-hydroxypyridin |
| 0,10 kg | 4-Hydroxypropylamino-3-nitrophenol |
| 0,15 kg | HC Yellow No.12 |
| 0,35 kg | 2-Methylresorcin |
| 1,20 kg | 2,5-Diaminotoluol Sulfat |
| 61,85 kg | Henna neutral |
| 23,00 kg | Natriumperborat |
| 5,00kg | Carboxymethylcellulose |

92,00 kg der vorstehenden Mischung werden in einem Mischer (zum Beispiel einem Lödige-Mischer) zusammengerührt und anschließend mit 8,00 kg Paraffinöl (35 mPa*s, gemessen bei 20 °C) besprüht.

### Herstellung eines Haarfärbemittelbreis

40 g des Haarfärbemittels werden mit 160 g warmen Wassers (40°-50°C) gut vermischt, um einen gut streichfähigen Brei zu erhalten. Dieses Gemisch, mit einem Pinsel aufgetragen, läßt man 30 Minuten bei 40°C auf hellbraunes Haar mit einem Grauanteil von 50% einwirken. Danach spült man den Haarfärbemittelbrei aus, shampooniert und trocknet das Haar. Das hellbraune Ausgangshaar mit einem Grauanteil von 50% zeigt einen einheitlichen hellen Tabakbraunton.

### Beispiel 7

### Haarfärbemittel in Pulverform

| | |
|---|---|
| 0,15 kg | Resorcin |
| 0,10 kg | 1-Hydroxy-3-aminobenzol |
| 0,15 kg | 2-Amino-3-hydroxypyridin |
| 0,15 kg | 4-Hydroxypropylamino-3-nitrophenol |
| 0,10 kg | HC Yellow No.12 |
| 0,30 kg | 2-Methylresorcin |
| 1,25 kg | 2,5-Diaminotoluol Sulfat |
| 65,80 kg | Henna neutral |
| 20,00 kg | Natriumperborat |
| 5,00kg | Carboxymethylcellulose |

93,00 kg der vorstehenden Mischung werden in einem Mischer (zum Beispiel einem Lödige-Mischer) zusammengerührt und anschließend mit 7,00 kg Paraffinöl (15 mPa*s, gemessen bei 20 °C) besprüht.

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf mittelblondes Haar mit einem Grauanteil von 50% einwirken. Danach spült man den Haarfärbemittelbrei aus, shampooniert und trocknet das Haar. Das mittelblonde Ausgangshaar mit einem Grauanteil von 50% zeigt nach der Behandlung einen einheitlichen Kastanienton.

### Beispiel 8

### Haarfärbemittel in Pulverform

| | |
|---|---|
| 0,30 kg | Resorcin |
| 0,10 kg | 1-Hydroxy-3-aminobenzol |
| 0,15 kg | 2-Amino-3-hydroxypyridin |
| 0,15 kg | HC Yellow No. 12 |
| 0,45 kg | 2-Methylresorcin |
| 2,00 kg | 2,5-Diaminotoluol Sulfat |
| 59,85 kg | Henna neutral |
| 22,00 kg | Natriumperborat |
| 5,00kg | Carboxymethylcellulose |

90,00 kg der vorstehenden Mischung werden in einem Mischer (zum Beispiel einem Lödige-Mischer) zusammengerührt und anschließend mit 10,00 kg Capryl-Caprinsäure Triglycerid besprüht.

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf mittelbraunes Haar mit einem Grauanteil von 50% einwirken. Danach spült man den Haarfärbemittelbrei aus, shampooniert und trocknet das Haar. Das mittelbraune Ausgangshaar mit einem Grauanteil von 50% weist nach der Behandlung einen einheitlichen dunklen Kakaoton auf.

### Beispiel 9

### Haarfärbemittel in Pulverform

| | |
|---|---|
| 0,50 kg | Resorcin |
| 0,20 kg | 1-Hydroxy-3-aminobenzol |
| 0,10 kg | 2-Amino-3-hydroxypyridin |
| 0,45 kg | 2-Methylresorcin |
| 2,25 kg | 2,5-Diaminotoluol Sulfat |
| 60,50 kg | Henna neutral |
| 22,00 kg | Natriumperborat |
| 5,00 kg | Carboxymethylcellulose |

91,00 kg der vorstehenden Mischung werden in einem Mischer (zum Beispiel einem Lödige-Mischer) zusammengerührt und anschließend mit 9,00 kg Octyldodecanol besprüht.

### Herstellung eines Haarfärbemittelbreis

40 g des Haarfärbemittels werden mit 160 g warmen Wassers (40°-50°C) gut vermischt, um einen gut streichfähigen Brei zu erhalten. Dieses Gemisch, mit einem Pinsel aufgetragen, läßt man 30 Minuten bei 40°C auf hellbraunes Haar mit einem Grauanteil von 50% einwirken. Danach spült man den Haarfärbemittelbrei aus, shampooniert und trocknet das Haar. Das hellbraune Ausgangshaar mit einem Grauanteil von 50% weist einen einheitlichen Mittelbraunton auf.

### Beispiel 10

### Haarfärbemittel in Pulverform

| | |
|---|---|
| 0,20 kg | Resorcin |
| 0,15 kg | 1-Hydroxy-3-aminobenzol |
| 0,01 kg | 4-Hydroxypropylamino-3-nitrophenol |
| 0,05 kg | 2-Amino-6-chlor-4-nitrophenol |
| 0,40 kg | 2-Methylresorcin |
| 0,90 kg | 2,5-Diaminotoluol Sulfat |
| 64,79 kg | Henna neutral |
| 21,00 kg | Natriumperborat |
| 5,00kg | Carboxymethylcellulose |

92,50 kg der vorstehenden Mischung werden in einem Mischer (zum Beispiel einem Lödige-Mischer) zusammengerührt und anschließend mit 7,50 kg Dimethyl-Polysiloxan besprüht.

### Herstellung eines Haarfärbemittelbreis

40 g des Haarfärbemittels werden mit 160 g warmen Wassers (40°-50°C) gut vermischt, um einen gut streichfähigen Haarfärbemittelbrei zu erhalten. Dieses Gemisch, mit einem Pinsel aufgetragen, läßt man 30 Minuten bei 40°C auf hellblondes Haar mit einem Grauanteil von 80% einwirken. Danach spült man den Haarfärbemittelbrei aus, shamponiert und trocknet das Haar. Das hellblonde Ausgangshaar mit einem Grauanteil von 80% liegt nach der Behandlung in einem einheitlichen Mittelblondton vor.

### Ergebnisse von vergleichenden Untersuchungen zum Nachweis der Erfindungshöhe

Es wurden orientierende Untersuchungen mit Haarfärbemitteln gemäß der Erfindung, die Henna neutral enthalten, durchgeführt. Als Vergleichs-Haarfärbemittel wurden Ansätze verwendet, die die der Zusammensetzung der zur Prüfung verwendeten Haarfärbemittel der Erfindung entsprechen, jedoch frei von Henna neutral sind.

Es hat sich eindeutig gezeigt, daß die mit den Haarfärbemitteln der Erfindung gefärbten Haare im Vergleich zu mit den entsprechenden Henna-neutral-freien Haarfärbemitteln behandelten Haaren bei den folgenden Untersuchungsmethoden überlegene Eigenschaften gezeigt haben, nämlich bei den Glanz-, den Naß- und Trocken-Kämmkraft- und den Volumenmessungen, Beurteilung des Griffs sowie bei thermoanalytischen Untersuchungen. Letztere zeigen für die mit den Haarfärbemitteln der Erfindung gefärbten Haare einen signifikant höheren Keratinschmelzpunkt im Vergleich zu den entsprechenden mit Henna-neutral-freien Haarfärbemitteln behandelten Haaren, was eindeutig eine Strukturstabilisierung durch Henna neutral belegt.

## Patentansprüche

1. Haarfärbemittel in Pulver- und/oder Granulatform auf Basis von Oxidationsfarbstoffvorprodukten, Oxidationsmitteln und direktziehenden Farbstoffen, von denen mindestens einer als natürlicher Pflanzenfarbstoff vorliegen muß, und Henna neutral sowie in Haarfärbemitteln üblichen Zusätzen.

2. Haarfärbemittel in Pulver- und/oder Granulatform auf Basis von Oxidationsfarbstoffvorprodukten, Oxidationsmitteln und direktziehenden Farbstoffen, in denen die direktziehenden Farbstoffe aus synthetischen und/oder pflanzlichen Farbstoffen bestehen, und Henna neutral sowie in Haarfärbemitteln üblichen Zusätzen.

3. Haarfärbemittel nach einem der Ansprüche 1 bis 2, in denen das pulverförmige Oxidationsmittel mit inerter nicht flüchtiger organischer Flüssigkeit und/oder bei etwa 35°C bis 50°C schmelzenden Wachsen oder wachsartigen inerten Stoffen umhüllt ist.

4. Haarfärbemittel nach einem der Ansprüche 1 bis 3, in denen das Oxidationsmittel Natriumperborat ist.

5. Haarfärbemittel nach einem der Ansprüche 1 bis 4, in denen die pflanzlichen Farbstoffe in isolierter, synthetischer oder in natürlicher Form als Pulver aus der Gruppe Henna rot, Henna schwarz, Walnußschalen, Indigo, Kamille, Blauholz, Faulbaumrinde einzeln oder im Gemisch enthalten sind.

6. Haarfärbemittel nach einem der Ansprüche 1 bis 5, in denen als direktziehende synthetische Farbstoffe 4-Hydroxypropylamino-3-nitrophenol, HC Red No. 3, N,N'-Bis-(2-hydroxyethyl)-2-nitro-p-phenylendiamin, HC Blue No. 2, HC Yellow No. 2, HC Yellow No. 12, HC Blue No. 12, 2-Amino-6-chlor-4-nitrophenol einzeln oder im Gemisch anwesend sind.

7. Haarfärbemittel nach einem der Ansprüche 1 bis 6, in denen die Oxidationsfarbstoffvorprodukte aus einer oder mehreren Entwicklersubstanz(en) sowie einer oder mehreren Kupplersubstanz(en) bestehen.

8. Haarfärbemittel nach Anspruch 7, in denen als Entwicklersubstanzen p-Phenylendiamin, p-Toluylendiamin, 2-Hydroxyethyl-1,4-diaminobenzol, p-Aminophenol, 3-Methyl-4-aminophenol und 2,4,5,6-Tetraaminopyrimidin einzeln oder im Gemisch enthalten sind.

9. Haarfärbemittel nach Anspruch 7 oder 8, in denen als Kupplersubstanzen α-Naphtol, Resorcin, 4-Chlorresorcin, 2-Methylresorcin, m-Aminophenol, m-Phenylendiamin, m-Toluylendiamin, 1,5- bzw. 1,7-Dihydroxynaphtalin, 5-Amino-2-methylphenol, 6-Amino-2-methylphenol einzeln oder im Gemisch enthalten sind.

10. Haarfärbemittel nach einem der Ansprüche 1 bis 9, in denen als Kombination Henna neutral und Tannine enthalten sind.

11. Haarfärbemittel nach einem der Ansprüche 1 bis 10, in denen der Gehalt an Oxidationsfarbstoffvorprodukten 1 bis 10 Gew.-% beträgt.

12. Haarfärbemittel nach einem der Ansprüche 1 bis 11, in denen der Gehalt an Oxidationsmittel 10 bis 35 Gew.-% beträgt.

13. Haarfärbemittel nach einem der Ansprüche 1 bis 12, in denen 5 bis 25 Gew-% inerte, nicht flüchtige, organische Flüssigkeit und/oder bei etwa 35 bis 50°C schmelzende Wachse oder wachsartige inerte Stoffe enthalten sind.

14. Haarfärbemittel nach Anspruch 13, in denen Paraffinöl und/oder Dimethyl-Polysiloxan, und /oder Capryl-Caprinsäure Triglycerid und/oder Octyldodecanol enthalten ist

15. Haarfärbemittel nach einem der Ansprüche 1 bis 14, in denen die enthaltenen Komponenten so abgestimmt vorliegen, daß nach Zugabe von Wasser der pH-Wert 5,0 bis 12,0 beträgt.

16. Haarfärbemittel nach einem der Ansprüche 1 bis 15, in denen 40 bis 65 Gew-% Henna neutral, bevorzugt 50 bis 60 Gew-% Henna neutral, enthalten sind.

17. Haarfärbemittelbrei, der ein Haarfärbemittel nach einem der Ansprüche 1 bis 16 in Mengen von 15 bis 25 Gew-% und 85 bis 75 Gew-% Wasser enthält.

18. Verfahren zur Herstellung eines Haarfärbemittels in Pulver-und/oder Granulatform nach einem der Ansprüche 1 bis 16, bei dem die Oxidationsfarbstoffvorprodukte, Oxidationsmittel und direktziehenden Farbstoffe in Pulverform unter Zugabe von in Haarfärbemitteln üblichen Zusätzen gut durchmischt werden und die erhaltene Pulvermischung unter ständiger Bewegung mit inerter, nicht flüchtiger organischer Flüssigkeit und /oder durch Erhitzen erhaltene flüssige Wachse oder wachsartige inerte Stoffe mit Schmelzbereich von etwa 35°C bis 50°C besprüht werden und das erhaltene Pulvergemisch gegebenenfalls in die Granulatform überführt wird.

## Claims

1. Powder-form or granular hair colorants based on oxidation dye precursors, oxidizing agents and substantive dyes of which at least one must be present as a natural vegetable dye, and henna neutral and additives typical of hair colorants.

2. Powder-form or granular hair colorants based on oxidation dye precursors, oxidizing agents and substantive dyes, in which the substantive dyes consist of synthetic and/or vegetable dyes, and henna neutral and additives typical of hair colorants.

3. Hair colorants as claimed in claim 1 or 2, in which the powder-form oxidizing agent is coated with inert nonvolatile organic liquid and/or waxes or wax-like inert materials melting at around 35 to 50°C.

4. Hair colorants as claimed in any of claims 1 to 3 in which the oxidizing agent is sodium perborate.

5. Hair colorants as claimed in any of claims 1 to 4 in which the vegetable dyes are present in isolated, synthetic or natural form as powders from the group consisting of henna red, henna black, walnut shells, indigo, camomile, logwood, black alder bark either individually or in admixture.

6. Hair colorants as claimed in any of claims 1 to 5, in which 4-hydroxypropylamino-3-nitrophenol, HC Red No. 3, N,N'-bis-(2-hydroxyethyl)-2-nitro-p-phenylenediamine, HC Blue No. 2, HC Yellow No. 2, HC Yellow No. 12, HC Blue No. 12, 2-amino-6-chloro-4-nitrophenol are present individually or in admixture as substantive synthetic dyes.

7. Hair colorants as claimed in any of claims 1 to 6, in which the oxidation dye precursors consist of one or more primary intermediate(s) and one or more secondary intermediate(s).

8. Hair colorants as claimed in claim 7, in which p-phenylenediamine, p-toluylenediamine, 2-hydroxyethyl-1,4-diaminobenzene, p-aminophenol, 3-methyl-4-aminophenol and 2,4,5,6-tetraaminopyrimidine are present individually or in admixture as primary intermediates.

9. Hair colorants as claimed in claim 7 or 8, in which α-naphthol, resorcinol, 4-chlororesorcinol, 2-methyl resorcinol, m-aminophenol, m-phenylenediamine, m-toluylenediamine, 1,5- or 1,7-dihydroxynaphthalene, 5-amino-2-methylphenol, 6-amino-2-methylphenol are present individually or in admixture as secondary intermediates.

10. Hair colorants as claimed in any of claims 1 to 9, in which henna neutral and tannins are present as a combination.

11. Hair colorants as claimed in any of claims 1 to 10, in which the content of oxidation dye precursors is 1 to 10% by weight.

12. Hair colorants as claimed in any of claims 1 to 11, in which the content of oxidizing agents is 10 to 35% by weight.

13. Hair colorants as claimed in any of claims 1 to 12, in which 5 to 25% by weight of inert nonvolatile organic liquid and/or waxes or wax-like materials melting at around 35 to 50°C are present.

14. Hair colorants as claimed in claim 13, in which paraffin oil and/or dimethyl polysiloxane and/or caprylic/capric acid triglyceride and/or octyldodecanol is/are present.

15. Hair colorants as claimed in any of claims 1 to 14, in which the components present are adapted to one another so that the pH value after the addition of water is in the range from 5.0 to 12.0.

16. Hair colorants as claimed in any of claims 1 to 15, in which 40 to 65% by weight of henna neutral and preferably 50 to 60% by weight of henna neutral is present.

17. A hair coloring paste containing the hair colorant claimed in any of claims 1 to 16 in quantities of 15 to 25% by weight and 85 to 75% by weight of water

18. A process for the production of the powder-form and/or granular hair colorant claimed in any of claims 1 to 16 in which the oxidation dye precursors, oxidizing agent and substantive dyes in powder form are thoroughly mixed while additives typical of hair colorants are added and the powder mixture obtained is sprayed while continuously agitated with inert, nonvolatile organic liquid and/or liquid waxes or wax-like inert materials obtained by heating with a melting range of about 35 to 50°C and the powder mixture obtained is optionally converted into granules.

## Revendications

1. Teintures pour cheveux sous forme pulvérulente et/ou granulaire à base de précurseur de colorant par oxydation, d'agents d'oxydation et de colorants à action directe, dont au moins un doit se présenter comme colorant végétal naturel, et à base de Henné neutre ainsi que des additifs usuels dans les teintures pour cheveux.

2. Teintures pour cheveux sous forme pulvérulente et/ou granulaire à base de précurseurs de colorants par oxydation, d'agents d'oxydation et de colorants à action directe consistant en des colorants synthétiques et/ou végétaux, et à base de Henné neutre, ainsi que d'additifs usuels dans les teintures pour les cheveux.

3. Teintures pour les cheveux selon l'une des revendications 1 à 2,
dans lesquelles
l'agent d'oxydation pulvérulent est enrobé d'un liquide organique non volatil inerte, et/ou de cires ou de substances inertes cireuses qui fondent à environ 35°C à 50°C.

4. Teintures pour les cheveux selon l'une des revendications 1 à 3,
dans lesquelles l'agent d'oxydation est le perborate de sodium.

5. Teintures pour les cheveux selon l'une des revendications 1 à 4,
dans lesquelles
les colorants végétaux sont contenus sous forme isolée, synthétique ou sous forme naturelle en tant que poudre choisie dans le groupe du Henné rouge, du Henné noir, des coques de noix, de l'indigo, de la camomille, du bois de Campèdre, des écorces de bourdaine, isolément ou en mélange.

6. Teintures pour les cheveux selon l'une des revendications 1 à 5,
dans lesquelles
comme colorants synthétiques à action directe le 4-hydroxy propylamino 3-nitrophénol, le rouge HC n-3, la NN'-bis (2-hydroxyéthyl) -2-nitro p-phenylènc diamine, le bleu HC n° 2, le jaune HC n° 2, le jaune HC n° 12, le bleu HC n° 12 et le 2-amino 6-chloro 4-nitrophénol sont présents isolément ou en mélange.

7. Teintures pour les cheveux selon l'une des revendications 1 à 6,
dans lesquelles
les produits précurseurs de colorant par oxydation consistent en une ou plusieurs substances de couplage ainsi qu'en un ou plusieurs substances de révélation.

8. Teintures pour les cheveux selon la revendication 7,
dans lesquelles
comme substance de révélation, la p-phénylène diamine, la p-toluylène diamine, le 2-hydroxyéthyl 1,4-diamino benzène, le p-aminophénol, le 3-méthyl 4-aminophénol, et la 2,4,5,6-tétra amino pyrimidinc, sont contenus isolément ou en mélange.

9. Teintures pour cheveux selon la revendication 7 ou la revendication 8,
dans lesquelles
comme substances de couplage, l'α-naphtol, le résorcinol, le 4-chloro résorcinol, le 2-méthyl résorcinol, le m-aminophénol, le m-phényléne diamine, la m-toluylène diamine, le 1,5 ou 1,7-dihydroxynaphtalène, le 5-amino 2-méthylphénol, le 6-amino 2-méthylphénol sont contenus isolément ou en mélange.

10. Teintures pour cheveux selon l'une des revendications 1 à 9,
dans lesquelles
en tant que combinaison, du Henné neutre et des tannins sont contenus.

11. Teintures pour les cheveux selon l'une des revendications 1 à 10,
dans lesquelles
la teneur en produits précurseurs de colorant par oxydation s'élève à 1 à 10 % en poids.

12. Teintures pour les cheveux selon l'une des revendications 1 à 11,
dans lesquelles
la teneur en agent d'oxydation s'élève à 10 à 35 % en poids.

13. Teintures pour les cheveux selon l'une des revendications 1 à 12,
dans lesquelles
de 5 à 25 % en poids de liquides inertes non volatils, organiques et/ou de cires qui fondent à environ 35 à 50°C ou des substances inertes cireuses sont contenues.

14. Teintures pour les cheveux selon la revendication 13,
dans lesquelles
de l'huile de paraffine et/ou du diméthylpolysiloxane et/ou un triglycéride d'acide caprique/caprinique et/ou de l'octyl dodécanol sont contenus.

15. Teintures pour les cheveux selon l'une des revendications 1 à 14,
dans lesquelles
les composants qui y sont contenus se présentent d'une manière coordonnée de sorte qu'après addition d'eau, la valeur du pH est de 5,0 à 12,0.

16. Teintures pour les cheveux selon l'une des revendications 1 à 15,
dans lesquelles
de 40 à 65 % en poids de Henné neutre, de préférence de 50 à 60 % en poids de Henné neutre sont contenus.

17. Pâte de teinture pour cheveux qui contient une teinture pour cheveux selon l'une des revendications 1 à 16 en quantité allant de 15 à 25 % en poids et de 85 à 75 % en poids d'eau.

18. Procédé de préparation d'une teinture pour cheveux sous forme pulvérulente et/ou granulaire, selon l'une des revendications 1 à 16, selon lequel
les produits précurseurs de colorant par oxydation, l'agent oxydant et les colorants à action directe sous forme pulvérulente, sont mélangés intimement par addition d'additifs usuels dans les teintures pour cheveux et le mélange de poudres obtenu est aspergé sous mouvement constant avec un liquide organique inerte non volatil, et/ou avec des cires liquides obtenues par chauffage ou des substances inertes cireuses ayant une zone de fusion d'environ 35°C à 50°C, et le mélange de poudre obtenu le cas échéant est converti en forme de granulé.
